# EUROPÄISCHE PATENTSCHRIFT

(11) **EP 0 617 952 B1**
(45) Veröffentlichungstag und Bekanntmachung des Hinweises auf die Patenterteilung: **29.10.1997**
(21) Anmeldenummer: 94102014.1
(22) Anmeldetag: 10.02.1994
(51) Int. Cl.: A61K 7/027, A61K 7/48

(54) **Kosmetische Stifte**
Cosmetic stick
Crayon cosmétique

(30) Priorität: 01.03.1993 DE 4306068
(43) Veröffentlichungstag der Anmeldung: 05.10.1994
(73) Patentinhaber: Beiersdorf Aktiengesellschaft, 20245 Hamburg (DE)
(72) Erfinder: Schneider, Günther, Dr., D-20251 Hamburg (DE); Teichmann, Stephan, Dr., Tairamachi Pound/A 302, Tokyo 152 (JP); Klier, Manfred, Dr., D-20521 Aumühle (DE); Aul, Marta, D-21244 Buchholz (DE)

(56) Entgegenhaltungen:
- WO-A-93/04658
- DE-A- 4 119 890
- FR-A- 2 417 980
- GB-A- 2 079 601
- DATABASE WPI Section Ch, Week 9010, Derwent Publications Ltd., London, GB; Class A10, AN 90-073603 & SU-A-1 503 817 (LENINGRAD FORESTRY ACAD. ET AL) 3. August 1989
- DATABASE WPI Section Ch, Week 9319, Derwent Publications Ltd., London, GB; Class A06, AN 93-157613 & SU-A-1 734 748 (AEROZOL SCI. PRODN ASSOC. ET AL) 23. Mai 1992
- CHEMICAL ABSTRACTS, vol. 100, no. 2, 9. Januar 1984, Columbus, Ohio, US; abstract no. 12459c, 'FATTY ESTERS AS BASES FOR COSMETICS' Seite 353 ;Spalte 1 ; & JP-A-58 162 510 (SHINEI KAGAKU KK ET AL) 27. September 1983
- CHEMICAL ABSTRACTS, Bd. 100, Nr. 24, 11 Juni 1984, Columbus, Ohio, US; Zusammenfassung Nr. 197641X, BOUTET D. 'NEW EMULSIFIERS USED IN COSMETICS' Seite 354; Spalte 1; & PARFUMS COSM. AROMES Bd. 54 , 1983 Seiten 49 - 53 BOUTET D.
- FIEDLER H. P. 'LEXICON DER HILFSTOFFE FÜR PHARMAZIE , KOSMETIK UND ANGRENZENDE GEBIETE' 1981 , CANTOR , AULENDORF, DE * Seite 175, Spalte 2 *

## Beschreibung

Die vorliegende Erfindung betrifft kosmetische Stifte, insbesondere Lippenstifte, bevorzugt Lippenpflegestifte, sowie Zusammensetzungen und Verfahren zur Herstellung kosmetischer Stifte.

Die Haut der Lippen besitzt nur eine äußerst dünne Hornschicht. Schweißdrüsen sind auf den Lippen gar nicht, Talgdrüsen nur vereinzelt zu finden. Daher ist die Lippenhaut praktisch frei von Fett und neigt, besonders bei kaltem und trockenem Wetter, zum Austrocknen. Dabei können sich kleine Risse in der Haut bilden, und die Empfindlichkeit der Lippen gegenüber chemischen, physikalischen und mikrobiellen Einwirkungen (z.B. Nahrungsmittel, Sonnenlicht, Herpes-Simplex-Viren) steigt.

Dies zu verhindern ist die Aufgabe von Lippenpflegestiften. Diese Produkte enthalten meist zu einem hohen Anteil Wachse und Fettkomponenten, die nach dem Auftragen eine abdeckende Schicht über den Lippen ausbilden.

In die Zubereitungen für Lippenpflegestifte können zusätzlich Wirkstoffe eingearbeitet werden, die der Lippenpflege oder dem Lippenschutz förderlich sind, z.B. Vitamine, Feuchtigkeit spendende Mittel, Lichtschutzmittel, abdeckende Pigmente usw.

Die Lederhaut der Lippen weist gut durchblutete Papillen auf, die bis dicht unter die Lippenoberfläche reichen. Daher sind die Lippen rötlich gefärbt und, je nach Teintfarbe der betreffenden Person, von der übrigen Gesichtshaut mehr oder weniger stark farblich abgesetzt. Ein Stilmittel der dekorativen Kosmetik ist dann auch, die Lippenfarbe durch entsprechende Kosmetika auf den Typ der Person abzustimmen.
Produkte dieser Art sind dekorative Lippenstifte, in welche verschiedenste Farbpigmente eingearbeitet werden können. Auch diese Stifte enthalten zu hohen Anteilen Wachse und Fettkomponenten, die nach dem Auftragen eine abdeckende Lipidschicht über den Lippen ausbilden.

Die Aufgabe dieser Schicht ist jedoch nicht vorderhand, die Lippenhaut vor dem Austrocknen zu schützen. Die Lipidschicht dient hier als auf den Lippen haftende Grundlage für die eingearbeiteten Pigmentstoffe; die Pigmente selbst können aus mancherlei Gründen nicht ohne eine solche Grundlage auf die Lippen aufgetragen werden.

Es ist auch möglich, die Eigenschaften der pflegenden und dekorativen Lippenstifte miteinander zu kombinieren, d.h., in dekorative Lippenstifte pflegende oder schützende Substanzen einzuarbeiten.

Technisch betrachtet sind fast alle Lippenstifte wasserfreie Fettmischungen aus festen oder halbfesten Wachsen und flüssigen Ölen, wobei die hochgereinigten Paraffinöle und -wachse die Lippenstiftgrundmasse darstellen.

Nach dem idealen Anforderungsprofil sollen sich Lippenstifte glatt und ohne großen Reibungswiderstand auftragen lassen. Außerdem soll ein Lippenstift schon bei leichtem Andruck einen nicht schmierigen, stumpfen oder klebrigen, aber dennoch gut haftenden Fettfilm an die Lippen abgeben. Durch diesen Fettfilm sollen die Lippen dann glatt und geschmeidig gemacht werden.

Darüber hinaus muß ein Lippenstift auch noch die Anforderungen erfüllen, daß er bruchfest und temperaturbeständig sein muß und nicht ausölen darf.

Übliche Grundstoffe des Standes der Technik sind
(1) flüssige Öle (z.B. Paraffinöle, Ricinusöl, Isopropylmyristat)
(2) halbfeste Bestandteile (z.B. Vaseline, Lanolin)
(3) feste Bestandteile (z.B. Bienenwachs, , Ceresin und Mikrokristalline Wachse bzw. Ozokerit)
(4) hochschmelzende Wachse (z.B. Camaubawachs, Candelillawachs)

Lippenstifte des Standes der Technik mit einem Gehalt an Paraffinen und Bienenwachs sind in "Kosmetik, Entwicklung Herstellung und Anwendung kosmetischer Mittel", S. 105 , Herausgeber: W.Umbach, Georg Thieme Verlag, Stuttgart - New York, 1988, beschrieben.

Der Stand der Technik hat aber eine Reihe von Nachteilen. Dazu zählt die Tatsache, daß wasserlösliche Wirkstoffe häufig nicht gut genug fettlöslich sind, als daß sie in nennenswertem Maße in die kosmetischen Grundlagen einzubauen wären. Andererseits wäre ein gewisser Wassergehalt durchaus erwünscht, um die Kompatibilität des kosmetischen Stiftes mit der menschlichen Haut zu erhöhen.

So ist aus dem DBP 23 35 549 ein Verfahren zur Herstellung eines kosmetischen Stiftes auf der Basis einer W/O-Emulsion bekannt. Nach dieser Lehre wird aus einer Polyhydroxyverbindung und einer nichtionogenen, oberflächenaktiven Verbindung ein Gel herstellt, dieses mit einer kosmetischen Grundlage vermischt und dann einen Gehalt an Wasser in die Mischung emulgiert.

Nach diesem Verfahren sind jedoch keine Stifte herzustellen, die über die gestellten universellen Anforderungen an einen kosmetischen Stift verfügen.

Ein weiterer Nachteil ist, daß Paraffinöle und -wachse bis zum gegenwärtigen Zeitpunkte unabdingbare Bestandteile für Lippenstifte waren. Obwohl es sich dabei um in guter Qualität erhältliche Rohstoffe handelt, und Stifte mit brauchbaren Eigenschaften mit ihrer Hilfe formuliert werden können, sind die Anwendungseigenschaften solcher kosmetischer Stifte jedoch begrenzt. Außerdem sind Paraffine wertvolle Grundstoffe, deren Vorkommen auf der Erde begrenzt sind. Die moderne Produktion geht in die Richtung nachwachsender Rohstoffe, also beispielsweise pflanzlicher Wachse oder Öle auf dem Gebiete der Kosmetik.

Es war jedoch bisher unmöglich, einen kosmetischen Stift auf der Basis der bekannten pflanzlichen Wachse, Fette oder Öle oder chemisch modifizierter pflanzlicher Wachse, Fette oder Öle zu konzipieren. Eine weiter Aufgabe der vorliegenden Erfindung war also, eine Grundlage für kosmetische Stifte, insbesondere Lippenstifte zu schaffen, welche auf mineralische Öle verzichten kann und stattdessen auf pflanzlichen oder gegebenenfalls tierischen Lipidkomponenten bzw. chemisch modifizierten Varianten davon basieren kann.

Es war überraschend und nicht vorhersehbar, daß kosmetische Stifte, frei von mineralischen Ölen, Fetten oder Wachsen, enthaltend
(a) Bienenwachs,
(b) Ester aus
   (ba) einer gesättigten Carbonsäure mit 10 - 19 Kohlenstoffatomen und
   (bb) einem gesättigten Alkohol mit 14 - 40 Kohlenstoffatomen,
(c) Wasser, sowie
(d) gegebenenfalls weitere Lipide und/oder übliche Hilfs- und Zusatzstoffe,
sowie
Kosmetische Stifte, frei von mineralischen Ölen, Fetten oder Wachsen,enthaltend
(a) Bienenwachs,
(b) Ester aus
   (ba) einer gesättigten Carbonsäure mit 20 - 40 Kohlenstoffatomen und
   (bb) einem gesättigten Alkohol mit 35 - 40 Kohlenstoffatomen
(c) Wasser, sowie
(d) gegebenenfalls weitere Lipide und/oder übliche Hilfs- und Zusatzstoffe,
Lippenstifte mit allen geforderten Eigenschaften ergeben würden, die die Nachteile des Standes der Technik beseitigen.

Zwar beschreibt die SU-A-1,734,748 Lippenstifte mit einem Gehalt an Bienenwachs und Stearylstearat, dies sind aber paraffinhaltig und konnten daher nicht den Weg zur vorliegenden Erfindung weisen.

Die vorliegende Erfindung ermöglicht, Emulsionslippenstifte zur Verfügung zu stellen, welche die Nachteile des Standes der Technik nicht aufweisen.

Ganz besonders vorteilhaft sind die Ester (A) - (J):
(A) Hexacosanylpalmitat (Synonym: Hexacosanylhexadecanoat) hat die Formel 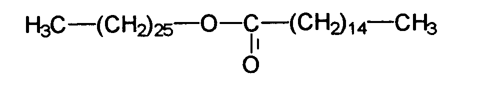
(B) Octacosanylpalmitat (Synonym: Octacosanylhexadecanoat) hat die Formel 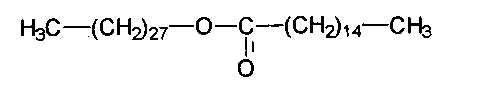
(C) Triacontanylpalmitat (Synonym: Triacontanylhexadecanoat) hat die Formel 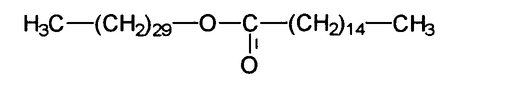
(D) Dotriacontanylpalmitat (Synonym: Dotriacontanhexadecanoat) hat die Formel 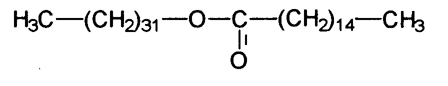
(E) Tetratriacontanylpalmitat (Synonym: Tetratriacontanhexadecanoat) hat die Formel 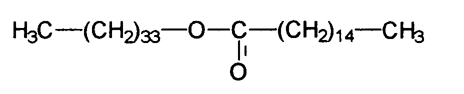
(F) Hexacosanylstearat (Synonym: Hexacosanyloctadecanoat) hat die Formel 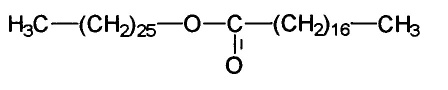
(G) Octacosanylstearat (Synonym: Octacosanyloctadecanoat) hat die Formel 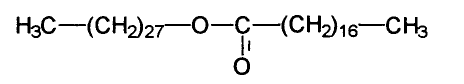
(H) Triacontanylstearat (Synonym: Triacontanyloctadecanoat) hat die Formel 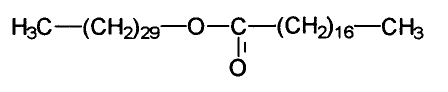
(I) Dotriacontanylstearat (Synonym: Dotriacontanoctahexadecanoat) hat die Formel 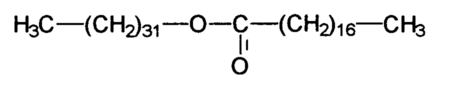
(J) Tetratriacontanylstearat (Synonym: Tetratriacontanyloctadecanoat) hat die Formel

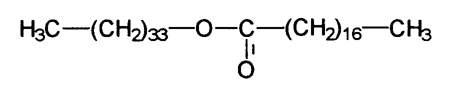

Der bevorzugte Ester ist das Octacosanylstearat.

Die Ester wurden von der Firma Koster Keunen Holland B.V. synthetisiert und zur Verfügung gestellt.

Herstellungsbedingt können die Ester gegebenenfalls gewisse unschädliche Nebenprodukte sowie nicht umgesetzte Ausgangssubstanzen enthalten. Vorteilhaft ist es, Handelsware einzusetzen, deren Gehalt an Ester mindestens 80 Gew.-%, bezogen auf das Gesamtgewicht des Produktes, enthält.

Bienenwachs (Synonyme sind: Cera flava (gelblich) und Cera alba (weiß), oder nach CTFA: Beeswax) besteht hauptsächlich aus Myricylpalmitoat, Cerotinsäure, Melissinsäure, höheren Alkoholen und Kohlenwasserstoffen. Neuere Untersuchungen legen nahe, daß Bienenwachs aus einer homologen Reihe von vornehmlich Hexadecanoatalkylestern zwischen C₃₅ und C₅₄ bestehen ("Lexikon der Hilfsstoffe für Pharmazie, Kosmetik und angrenzende Gebiete", H.P. Fiedler, 3. Aufl., 1989, Editio Cantor Aulendorf, und dort unter dem Stichwort "Bienenwachs" zitierte Quellen und Querverweise).

Bienenwachs ist seit alters ein wichtiger kosmetischer Bestandteil, insbesondere für Crèmes und Salbenzubereitungen, aber auch für kosmetische Stifte. Ein Lippenstift des Standes der Technik enthält 4,0 Gew.-% Bienenwachs.

Zu hohe Anteile an Bienenwachs haben bisher immer dazu geführt, daß kosmetische Stifte glanzlos und körnig wurden. Auch die Stabilität solcher Stifte ließ ständig zu wünschen übrig. Es war immer ein erheblicher Nachteil, daß auf Bienenwachs basierende kosmetische Stifte kosmetisch unelegant waren.

Eine gewisse Menge Bienenwachs allerdings verleiht Lippenstiften bekanntermaßen Festigkeit und bewirkt, daß die Lippenstiftmasse gut auf den Lippen haftet ("Kosmetik, Entwicklung, Herstellung und Anwendung kosmetischer Mittel", S. 104 und passim, W. Umbach (Herausgeber), 1988, Georg Thieme Verlag, Stuttgart). Gleichwohl ist Bienenwachs, wohl aus den obenstehenden Erwägungen, kein obligatorischer Bestandteil kosmetischer Stifte.

Vorteilhaft enthalten die erfindungsgemäßen kosmetischen Stifte
- 0,5 - 50 Gew.-%: Bienenwachs
- 0,5 - 50 Gew.-%: eines Esters aus einer gesättigten Carbonsäure mit 10 - 19 Kohlenstoffatomen und einem gesättigten Alkohol mit 14 - 40 Kohlenstoffatomen
- 0,1 - 20 Gew.-%: Wasser,
insbesondere
- 2,0 - 20 Gew.-%: Bienenwachs
- 2,0 - 25 Gew.-%: eines Esters aus einer gesättigten Carbonsäure mit 10 - 19 Kohlenstoffatomen und einem gesättigten Alkohol mit 14 - 40 Kohlenstoffatomen
- 1,0 - 10 Gew.-%: Wasser,
bevorzugt
- 3,0 - 10 Gew.-%: Bienenwachs
- 5,0 - 20 Gew.-%: eines Esters aus einer gesättigten Carbonsäure mit 10 - 19 Kohlenstoffatomen und einem gesättigten Alkohol mit 14 - 40 Kohlenstoffatomen
- 1,0 - 5 Gew.-%: Wasser.
ganz besonders bevorzugt
- 4,0 - 6,0 Gew.-%: Bienenwachs
- 15,0 - 18,0 Gew.-%: eines Esters aus einer gesättigten Carbonsäure mit 10 - 19 Kohlenstoffatomen und einem gesättigten Alkohol mit 14 - 40 Kohlenstoffatomen
- 2,0 - 3,0 Gew.-%: Wasser

Es ist bevorzugt, das Verhältnis von Bienenwachs und einem oder mehreren Estern aus einer gesättigten Carbonsäure mit 10 - 19 Kohlenstoffatomen und einem gesättigten Alkohol mit 14 - 40 Kohlenstoffatomen insbesondere den Estern (A) - (J), besonders bevorzugt Octacosanylstearat, im Verhältnis von etwa 1 : 1 bis etwa 1 : 5, insbesondere etwa 1 : 3 zu wählen.

Vorteilhaft enthalten die erfindungsgemäßen kosmetischen Stifte ferner
- 0,5 - 50 Gew.-%: Bienenwachs
- 0,5 - 50 Gew.-%: eines Esters aus einer gesättigten Carbonsäure mit 20 - 40 Kohlenstoffatomen und einem gesättigten Alkohol mit 35 - 40 Kohlenstoffatomen
- 0,1 - 20 Gew.-%: Wasser,
insbesondere
- 2,0 - 20 Gew.-%: Bienenwachs
- 2,0 - 25 Gew.-%: eines Esters aus einer gesättigten Carbonsäure mit 20 - 40 Kohlenstoffatomen und einem gesättigten Alkohol mit 35 - 40 Kohlenstoffatomen
- 1,0 - 10 Gew.-%: Wasser,
bevorzugt
- 3,0 - 10 Gew.-%: Bienenwachs
- 5,0 - 20 Gew.-%: eines Esters aus einer gesättigten Carbonsäure mit 20 - 40 Kohlenstoffatomen und einem gesättigten Alkohol mit 35 - 40 Kohlenstoffatomen
- 1,0 - 5 Gew.-%: Wasser.
ganz besonders bevorzugt
- 4,0 - 6,0 Gew.-%: Bienenwachs
- 15,0 - 18,0 Gew.-%: eines Esters aus einer gesättigten Carbonsäure mit 20 - 40 Kohlenstoffatomen und einem gesättigten Alkohol mit 35 - 40 Kohlenstoffatomen
- 2,0 - 3,0 Gew.-%: Wasser

Es ist bevorzugt, das Verhältnis von Bienenwachs und einem oder mehreren Estern aus einer gesättigten Carbonsäure mit 20 - 40 Kohlenstoffatomen und einem gesättigten Alkohol mit 35 - 40 Kohlenstoffatomen, im Verhältnis von etwa 1 : 1 bis etwa 1 : 5, insbesondere etwa 1 : 3 zu wählen.

Die Emulsionslippenstifte enthalten also eine gewisse Menge Wasser. Es ist zwar möglich, auf einen Emulgator zu verzichten, da Bienenwachs selbst Emulgatoreigenschaften hat. Vorteilhaft und bevorzugt ist jedoch, einen Emulgator einzuarbeiten, insbesondere einen Wasser-in-Öl-Emulgator (W/O-Emulgator).

Als besonders bevorzugte W/O-Emulgatoren, die zu überraschend stabilen kosmetischen Stiften mit besonders guter Hautfreundlichkeit führen und darüber hinaus kosmetisch besonders elegante Rezepturen ergeben, haben sich überraschend die Polyglycerinfettsäureester erwiesen. Besonders vorteilhaft ist das Polyglyceryl-3-diisostearat (Synonym: Triglyceryldiisostearat).

Die erfindungsgemäß vorteilhaften Polyglycerinester, deren Vorkommen, Eigenschaften und Verwendung in der Kosmetik sind aufgezählt und abgehandelt im "Lexikon der Hilfsstoffe für Pharmazie, Kosmetik und angrenzende Gebiete", H.P. Fiedler, 3. Aufl., 1989, Editio Cantor Aulendorf, dort unter den Stichwörtern "Polyglycerinester", "Polyglycerolester", "Polyglyceryl-3 diisostearate", "Triglycerin" sowie "Triglyceryldiisostearat" usw. zitierte Quellen und Querverweise. Die dort beschriebenen Polyglycerinester sind für die vorliegende Erfindung gut geeignet.

Erfindungsgemäß sind also kosmetische Stifte, welche außer durch einen Gehalt an Bienenwachs, Octacosanylstearat und Wasser dadurch gekennzeichnet sind, daß sie einen oder mehrere Emulgatoren aus der Gruppe der Polyglycerinfettsäureester, insbesondere das Polyglyceryl-3-diisostearat enthalten.

Die Emulgatoren können vorteilhaft in Gehalten von 0,05 - 10,0 Gew.-% in die erfindungsgemäßen Zusammensetzungen eingearbeitet werden, insbesondere in Gehalten von 1,00 - 8,00 Gew.-%, besonders bevorzugt 2,00 - 4,00 Gew.-%.

In die erfindungsgemäßen Zubereitungen können vorteilhaft die üblichen Bestandteile kosmetischer Stifte eingearbeitet werden, also Wachse, insbesondere pflanzliche und/oder tierische Wachse oder chemisch modifizierte Darivate davon, insbesondere Camaubawachs, Candelillawachs und dergleichen, Kohlenwasserstoffe, Fette und Öle für die Grundsubstanz, sowie die üblichen Hilfs- und Zusatzstoffe wie Parfümöle, Konservierungsmittel, Farbpigmente, Lichtschutzmittel, Stabilisatoren.

Zusätzlich können Pflegewirkstoffe eingearbeitet werden, welche sich nicht wie bisher auf die fettlöslichen Wirkstoffe beschränken, sondern auch aus der Gruppe der wasserlöslichen Wirkstoffe gewählt werden können, beispielsweise Vitamine und dergleichen mehr.

Es ist insbesondere von Vorteil, den an 100 Gew.-% fehlenden Rest der Stiftmasse aus der Gruppe der folgenden Stoffe zu wählen:

Glycerintricarbonsäureester (Synonym: Triglyceride), Guerbet-Alkohole, Myristylmyristat, Jojobaöl und verwandte Substanzen. Insbesondere können vorteilhaft weitere flüssige Fettkomponenten in die erfindungsgemäßen Zusammensetzungen eingearbeitet werden, dazu zählen z.B. fraktionierte Kokosöle.

Auch hier können vorteilhaft zusätzliche Hilfs- und Zusatzstoffe sowie Wirkstoffe, insbesondere Pflegewirkstoffe eingearbeitet werden, welche sich nicht wie bisher auf die fettlöslichen Wirkstoffe beschränken, sondern auch aus der Gruppe der wasserlöslichen Wirkstoffe gewählt werden können, beispielsweise Vitamine und dergleichen mehr.

Erfindungsgemäß sind also auch Zusammensetzungen für kosmetische Stifte, enthaltend
(a) Bienenwachs
(b) Octacosanylstearat und
(c) Wasser
   und Stoffen, gewählt aus der Gruppe
(d) Glycerintricarbonsäureester
(e) Guerbet-Alkohole
(f) Myristylmyristat
(g) Jojobaöl
(h) fraktionierte Kokosöle,
und gegebenenfalls zusätzlich Stoffen, gewählt aus der Gruppe der Wachse, Kohlenwasserstoffe, Fette, Öle, weitere Hilfs- und Zusatzstoffe wie Parfümöle, Konservierungsmittel, Farbpigmente, Lichtschutzmittel, Stabilisatoren sowie der fettlöslichen und/oder wasserlöslichen Wirkstoffe.

Es ist allerdings, aus den zuvor angegebenen Gründen vorteilhaft, auf Kohlenwasserstoffe gänzlich zu verzichten. Nichtsdestoweniger ergeben die erfindungsgemäßen Zusammensetzungen auch zusammen mit Kohlenwasserstoffen äußerst anspruchsvolle kosmetische Stifte mit vorteilhaften Eigenschaften.

Die Glycerintriester können vorteilhaft aus der Gruppe der sogenannten Neobee-Oils und der Fette gewählt werden. Zum Stande der Technik der Glycerintriester, deren Vorkommen, Eigenschaften und Verwendung in der Kosmetik siehe "Lexikon der Hilfsstoffe für Pharmazie, Kosmetik und angrenzende Gebiete", H.P. Fiedler, 3. Aufl., 1989, Editio Cantor Aulendorf, dort unter dem Stichwort "Triglyceride" zitierte Quellen und Querverweise. Die dort beschriebenen Triglyceride sind für die vorliegende Erfindung gut geeignet.

Unter Guerbet-Alkoholen werden verzweigtkettige Alkohole des allgemeinen Schemas 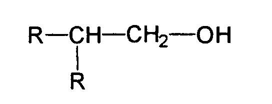 verstanden. R stellt dabei bevorzugt Kohlenwasserstoffreste von C₆ bis C₁₂ dar (Zum Stande der Technik der Herstellung, Eigenschaften und Verwendung der Guerbet-Alkohole: "Lexikon der Hilfsstoffe für Pharmazie, Kosmetik und angrenzende Gebiete", H.P. Fiedler, 3. Aufl., 1989, Editio Cantor Aulendorf, dort unter dem Stichwort "Guerbet-Alkohole" zitierte Quellen und Querverweise).

Obwohl es denkbar ist und gegebenenfalls vorteilhaft sein kann, Stifte gemäß der vorliegenden Erfindung auch für andere Anwendungen denn als Lippenstifte zu konzipieren, beispielsweise als Deodorantstifte ("Sticks"), ist in den betreffenden Anwendungsgebieten mit vergleichsweise geringer Verbraucherakzeptanz zu rechnen, da die erfindungsgemäßen Massen zumeist leicht fettig wirken. Letzteres ist bei einem Lippenstift auch durchaus erwünscht und sehr angenehm.

Von einem Deodorantstift wird jedoch, jedenfalls nach Einschätzung des mitteleuropäischen Verbraucherspektrums, erwartet, daß er eben nicht fettet.

Die erfindungsgemäßen kosmetischen Stifte lassen sich vorteilhaft erzeugen nach einem Verfahren , dadurch gekennzeichnet, daß man
(1) Bienenwachs und Octacosanylstearat,
   sowie gegebenenfalls einen oder mehrere Emulgatoren, gewählt aus der Gruppe der W/O-Emulgatoren, insbesondere der Polyglycerinfettsäureester, besonders vorteilhaft: Polyglyceryl-3-diisostearat
   sowie Fettkomponenten, gewählt aus der Gruppe der Glycerintricarbonsäureester, Guerbet-Alkohole, Myristylmyristat, Jojobaöl und/oder verwandter Substanzen und der fraktionierten Kokosöle
   aufschmilzt und
(2) kontinuierlich Wasser hinzugibt, bis der gewünschte Wassergehalt des kosmetischen Stiftes erreicht ist,
(3) während der Wasserzugabe die entstandene Mischung verrührt,
(4) und die gleichmäßige verrührte Mischung dann in Gußformen einbringt und langsam abkühlen läßt.

In den nachfolgenden Beispielen soll die vorliegende Erfindung beschrieben werden. Die einzelnen Einsatzstoffe sind in Analogie zur CTFA-Nomenklatur gewählt, außer bei solchen Substanzen, in welchen eine einfache chemische Bezeichnung existiert (z.B: Wasser).

### Beispiel 1

| | Gew.-% |
|---|---|
| Caprylic/Capric Triglyceride | 25 |
| Octyldodecanol | 25 |
| Caprylic/Capric Diglyceryl Succinate | 5 |
| Jojoba Öl | 5 |
| Myristylmyristat | 10 |
| Octacosanylstearat | 20 |
| Bienenwachs weiß | 9 |
| Octyl Methoxycinnamat | 1 |

### Beispiel 2

| | Gew.-% |
|---|---|
| Octyldodecanol | 45 |
| Caprylic/Capric Diglyceryl Succinate | 10 |
| Squalan | 7 |
| Jojoba Öl | 3 |
| Myristylmyristat | 8 |
| Carnauba Wachs | 2 |
| Octacosanylpalmitat | 11 |
| Octacosanylstearat | 4 |
| Beenenwachs weiß | 10 |

### Beispiel 3

| | Gew.-% |
|---|---|
| Cetylpalmitat | 8 |
| Squalan | 15 |
| Weizenkeimöl | 5 |
| Propylenglycoldicaprylat/caproat | 10 |
| Caprylic/Capric Triglyceride | 10 |
| Octyldodecanol | 20 |
| Polyisobuten | 2 |
| Cyclomethicone | 10 |
| Hexacosanylpalmitat | 10 |

### Beispiel 4

| | Gew.-% |
|---|---|
| Bienenwachs weiß | 26 |
| Caprylic/Capric Diglyceryl Succinate | 15 |
| Propylenglycol Dicaprylate/Dicaprate | 7 |
| Caprylic/Capric Triglyceride | 24 |
| Squalan | 10 |
| Schibutter | 7 |
| Octacosanylstearat | 6 |
| Octylmethoxycinnamat | 5 |

### Beispiel 5

| | Gew.-% |
|---|---|
| Caprylic/Capric Triglyceride | 14 |
| Octyldodecanol | 20 |
| Jojoba Öl | 10 |
| Myristylmyristat | 10 |
| Octacosanylstearat | 20 |
| Cetylpalmitat | 2 |
| Bienenwachs weiß | 8 |
| Polyglyceryl-3-diisostearat | 4 |
| Glycin | 1 |
| ZnSO₄ * H₂O | 1 |
| Wasser (der pH wird mit NaOH auf 6 - 7 eingestellt) | 10 |

### Beispiel 6

| | Gew.-% |
|---|---|
| Caprylic/Capric Triglyceride | 20 |
| Octyldodecanol | 15 |
| Cetearylalkohol | 5 |
| Schibutter | 10 |
| Jojoba Öl | 8 |
| Polyglyceryl-3-diisostearat | 2 |
| Polyisobuten | 2 |
| Octacosanylstearat | 10 |
| Cetylpalmitat | 5 |
| Bienenwachs weiß | 10 |
| Glycerin | 3 |
| Weizenkeimöl | 2 |
| Triacontanylpalmitat | 5 |
| Wasser | 3 |

### Beispiel 7

| | Gew.-% |
|---|---|
| Octyldodecanol | 42 |
| Caprylic/Capric Diglyceryl Succinate | 5 |
| Squalan | 5 |
| Jojoba Öl | 5 |
| Myristylmyristat | 8 |
| Octacosanyl Stearat | 20 |
| Bienenwachs weiß | 10 |
| Polyglyceryl-3-Diisostearat | 2 |
| Wasser | 3 |

### Beispiel 8

| | Gew.-% |
|---|---|
| Caprylic/Capric Triglyceride | 25 |
| Octyldodecanol | 11 |
| Caprylic/Capric Diglyceryl Succinate | 12,5 |
| Propylenglycol Dicaprylat/Dicaprat | 7 |
| Bienenwachs weiß | 26 |
| Polyglyceryl-3-Oleat | 3,5 |
| Octacosanylpalmitat | 5 |
| Wasser | 10 |

### Beispiel 9

| | Gew.-% |
|---|---|
| Jojoba Öl | 4 |
| Myristylmyristat | 7 |
| Polyglyceryl-3-oleat | 4 |
| Glyceryllanolat | 1,5 |
| Wollwachsalkohol | 1 |
| Octacosanylstearat | 5 |
| Ceresin | 15 |
| Caprylic/Capric Diglyceryl Succinate | 12,5 |
| Propylenglycol Dicaprylat/Dicaproat | 7 |
| Caprylic Capric Triglyceride | 2,5 |
| Octyldodecanol | 33 |
| Cetearylalkohol | 0,5 |
| Tocopherylacetat | 0.1 |
| Wasser | 4,9 |

### Beispiel 10

Die kosmetischen Stifte gemäß den Beispielen 1 - 9 wurden anhand subjektiver und objektiver Bewertungskriterien beurteilt.

Subjektive Bewertungskriterien waren das Gefühl beim Auftragen (stumpf, geschmeidig, fettig, schmierig, klebrig), der optische Eindruck (matt, glänzend,). In allen Fällen zeichneten sich die erfindungsgemäßen kosmetischen Stifte durch überlegenes Auftragegefühl und ausgezeichneten optischen Eindruck aus.

Objektive Bewertungskriterien waren Bruchstabilität, Masseabrieb, Penetration.

Im Vergleich mit Zusammensetzungen des Standes der Technik wiesen die erfindungsgemäßen Zusammensetzungen eine verbesserte Bruchstabilität auf.

Der Masseabrieb ist leicht zu ermitteln, indem ein Stift mit einer definierten Andruckkraft auf einer Unterlage entlanggezogen wird. Stifte mit niedrigem Masseabrieb werden als trocken, Stifte mit hohem Masseabrieb als schmierig empfunden.

Im Vergleich mit Zusammensetzungen des Standes der Technik wiesen die erfindungsgemäßen Zusammensetzungen einen verbesserten Masseabrieb auf. Die Masseabriebswerte liegen in dem Bereich, der mit einem sehr angenehmen Anwendungsgefühl verbunden wird.

Die Penetration ist zu ermitteln, indem die Eindringtiefe einer spitzen Nadel in die Lippenstiftmasse gemessen wird. Stiftmassen, bei welchen die Nadel kaum eindringt, werden als hart empfunden, solche, bei welchen die Nadel sehr leicht eindringt wird ebenfalls leicht unangenehm, weil zu weich empfunden.

Im Vergleich mit Zusammensetzungen des Standes der Technik wiesen die erfindungsgemäßen Zusammensetzungen eine verbesserte Penetration auf. Die Penentrationswerte liegen in dem Bereich, der mit einem sehr angenehmen Anwendungsgefühl verbunden wird.

## Patentansprüche

1. Kosmetische Stifte, frei von mineralischen Ölen, Fetten oder Wachsen, enthaltend
(a) Bienenwachs,
(b) Ester aus
(ba) einer gesättigten Carbonsäure mit 10 - 19 Kohlenstoffatomen und
(bb) einem gesättigten Alkohol mit 14 - 40 Kohlenstoffatomen,
(c) Wasser, sowie
(d) gegebenenfalls weitere Lipide und/oder übliche Hilfs- und Zusatzstoffe.

2. Kosmetische Stifte, frei von mineralischen Ölen, Fetten oder Wachsen,enthaltend
(a) Bienenwachs,
(b) Ester aus
(ba) einer gesättigten Carbonsäure mit 20 - 40 Kohlenstoffatomen und
(bb) einem gesättigten Alkohol mit 35 - 40 Kohlenstoffatomen
(c) Wasser, sowie
(d) gegebenenfalls weitere Lipide und/oder übliche Hilfs- und Zusatzstoffe.

3. Kosmetische Stifte nach Anspruch 1, dadurch gekennzeichnet, daß sie
0,5 - 50 Gew.-% Bienenwachs
0,5 - 50 Gew.-% eines Esters aus einer gesättigten Carbonsäure mit 10 - 19 Kohlenstoffatomen und einem gesättigten Alkohol mit 14 - 40 Kohlenstoffatomen
0,1 - 20 Gew.-% Wasser,
insbesondere
2,0 - 20 Gew.-% Bienenwachs
2,0 - 25 Gew.-% eines Esters aus einer gesättigten Carbonsäure mit 10 - 19 Kohlenstoffatomen und einem gesättigten Alkohol mit 14 - 40 Kohlenstoffatomen
1,0 - 10 Gew.-% Wasser,
bevorzugt
3,0 - 10 Gew.-% Bienenwachs
5,0 - 20 Gew.-% eines Esters aus einer gesättigten Carbonsäure mit 10 - 19 Kohlenstoffatomen und einem gesättigten Alkohol mit 14 - 40 Kohlenstoffatomen
1,0 - 5 Gew.-% Wasser.
ganz besonders bevorzugt
4,0 - 6,0 Gew.-% Bienenwachs
15,0 - 18,0 Gew.-% eines Esters aus einer gesättigten Carbonsäure mit 10 - 19 Kohlenstoffatomen und einem gesättigten Alkohol mit 14 - 40 Kohlenstoffatomen
2,0 - 3,0 Gew.-% Wasser
enthalten.

4. Kosmetische Stifte nach Anspruch 2, dadurch gekennzeichnet, daß sie
0,5 - 50 Gew.-% Bienenwachs
0,5 - 50 Gew.-% eines Esters aus einer gesättigten Carbonsäure mit 20 - 40 Kohlenstoffatomen und einem gesättigten Alkohol mit 35 - 40 Kohlenstoffatomen
0,1 - 20 Gew.-% Wasser,
insbesondere
2,0 - 20 Gew.-% Bienenwachs
2,0 - 25 Gew.-% eines Esters aus einer gesättigten Carbonsäure mit 20 - 40 Kohlenstoffatomen und einem gesättigten Alkohol mit 35 - 40 Kohlenstoffatomen
1,0 - 10 Gew.-% Wasser,
bevorzugt
3,0 - 10 Gew.-% Bienenwachs
5,0 - 20 Gew.-% eines Esters aus einer gesättigten Carbonsäure mit 20 - 40 Kohlenstoffatomen und einem gesättigten Alkohol mit 35 - 40 Kohlenstoffatomen
1,0 - 5 Gew.-% Wasser.
ganz besonders bevorzugt
4,0 - 6,0 Gew.-% Bienenwachs
15,0 - 18,0 Gew.-% eines Esters aus einer gesättigten Carbonsäure mit 10 - 19 Kohlenstoffatomen und einem gesättigten Alkohol mit 35 - 40 Kohlenstoffatomen
2,0 - 3,0 Gew.-% Wasser
enthalten.

5. Kosmetische Stifte nach Anspruch 1 oder 2, dadurch gekennzeichnet, daß sie Bienenwachs und einen oder mehrere Ester aus einer gesättigten Carbonsäure mit 10 - 19 Kohlenstoffatomen und einem gesättigten Alkohol mit 14 - 40 Kohlenstoffatomen bzw. einen oder mehrere Ester aus einer gesättigten Carbonsäure mit 20 - 40 Kohlenstoffatomen und einem gesättigten Alkohol mit 35 - 40 Kohlenstoffatomen im Verhältnis von etwa 1 : 1 bis etwa 1 : 5, insbesondere etwa 1 : 3 enthalten.

6. Kosmetische Stifte nach Anspruch 1 oder 2, dadurch gekennzeichnet daß der oder die Ester gewährt werden aus der Gruppe
Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat.

7. Kosmetische Stifte nach Anspruch 1 oder 2, dadurch gekennzeichnet, daß sie einen oder mehrere Emulgatoren aus der Gruppe der Polyglycerinfettsäureester, insbesondere das Polyglyceryl-3-diisostearat, enthalten.

8. Kosmetische Stifte nach Anspruch 1 oder 2, dadurch gekennzeichnet, daß sie neben den Komponenten
(a) Bienenwachs
(b) Octacosanylstearat und
(c) Wasser
außerdem Fettkomponenten, gewählt aus der Gruppe
(d) Glycerintricarbonsäureester
(e) Guerbet-Alkohole
(f) Myristylmyristat
(g) Jojobaöl
(h) fraktionierte Kokosöle,
enthalten.

9. Kosmetische Stifte nach Anspruch 1 oder 2, dadurch gekennzeichnet, daß sie neben den Komponenten der Gruppe
(a) Bienenwachs
(b) Octacosanylstearat und
(c) Wasser
und gegebenenfalls
Fettkomponenten, gewählt aus der Gruppe
(d) Glycerintricarbonsäureester
(e) Guerbet-Alkohole
(f) Myristylmyristat
(g) Jojobaöl
(h) fraktionierte Kokosöle,
zusätzlich Stoffe, gewählt aus der Gruppe Wachse, Kohlenwasserstoffe, Fette, Öle, Hilfs- und Zusatzstoffe wie Parfümöle, Konservierungsmittel, Farbpigmente, Lichtschutzmittel, Stabilisatoren sowie der fettlöslichen und/oder wasserlöslichen Wirkstoffe enthalten.

10. Verfahren zur Herstellung von kosmetischen Stiften nach einem der Ansprüche 1 - 9, dadurch gekennzeichnet, daß man
(1) Bienenwachs und Octacosanylstearat,
sowie gegebenenfalls einen oder mehrere Emulgatoren,gewählt aus der Gruppe der W/O-Emulgatoren, insbesondere der Polyglycerinfettsäureester, besonders vorteilhaft: Polyglyceryl-3-diisostearat
sowie Fettkomponenten, gewählt aus der Gruppe der Glycerintricarbonsäureester, Guerbet-Alkohole, Myristylmyristat, Jojobaöl und/oder verwandter Substanzen und der fraktionierten Kokosöle
aufschmilzt und gegebenenfalls den Schritten (2) und (3) unterwirft, nämlich
(2) kontinuierlich Wasser hinzugibt, bis der gewünschte Wassergehalt des kosmetischen Stiftes erreicht ist,
(3) während der Wasserzugabe die entstandene Mischung verrührt,
(4) und die gleichmäßige verrührte Mischung dann in Gußformen einbringt und langsam abkühlen läßt.

## Claims

1. Cosmetic sticks, free from mineral oils, fats or waxes, containing
(a) beeswax,
(b) esters of
(ba) a saturated carboxylic acid having 10-19 carbon atoms and
(bb) a saturated alcohol having 14-40 carbon atoms,
(c) water and
(d) optionally other lipids and/or customary auxiliaries and additives.

2. Cosmetic sticks, free from mineral oils, fats or waxes, containing
(a) beeswax,
(b) esters of
(ba) a saturated carboxylic acid having 20-40 carbon atoms and
(bb) a saturated alcohol having 35-40 carbon atoms,
(c) water and
(d) optionally other lipids and/or customary auxiliaries and additives.

3. Cosmetic sticks according to Claim 1, characterized in that they contain
0.5 - 50% by weight of beeswax,
0.5 - 50% by weight of an ester of a saturated carboxylic acid having 10-19 carbon atoms and a saturated alcohol having 14-40 carbon atoms, and
0.1 - 20% by weight of water,
in particular
2.0 - 20% by weight of beeswax,
2.0 - 25% by weight of an ester of a saturated carboxylic acid having 10-19 carbon atoms and a saturated alcohol having 14-40 carbon atoms, and
1.0 - 10% by weight of water,
preferably
3.0 - 10% by weight of beeswax,
5.0 - 20% by weight of an ester of a saturated carboxylic acid having 10-19 carbon atoms and a saturated alcohol having 14-40 carbon atoms, and
1.0 - 5% by weight of water,
very particularly preferably
4.0 - 6.0% by weight of beeswax,
15.0 - 18.0% by weight of an ester of a saturated carboxylic acid having 10-19 carbon atoms and a saturated alcohol having 14-40 carbon atoms, and
2.0 - 3.0% by weight of water.

4. Cosmetic sticks according to Claim 2, characterized in that they contain
0.5 - 50% by weight of beeswax,
0.5 - 50% by weight of an ester of a saturated carboxylic acid having 20-40 carbon atoms and a saturated alcohol having 35-40 carbon atoms, and
0.1 - 20% by weight of water,
in particular
2.0 - 20% by weight of beeswax,
2.0 - 25% by weight of an ester of a saturated carboxylic acid having 20-40 carbon atoms and a saturated alcohol having 35-40 carbon atoms, and
1.0 - 10% by weight of water,
preferably
3.0 - 10% by weight of beeswax,
5.0 - 20% by weight of an ester of a saturated carboxylic acid having 20-40 carbon atoms and a saturated alcohol having 35-40 carbon atoms, and
1.0 - 5% by weight of water,
very particularly preferably
4.0 - 6.0% by weight of beeswax,
15.0 - 18.0% by weight of an ester of a saturated carboxylic acid having 10-19 carbon atoms and a saturated alcohol having 35-40 carbon atoms, and
2.0 - 3.0% by weight of water.

5. Cosmetic sticks according to Claim 1 or 2, characterized in that they contain beeswax and one or more esters of a saturated carboxylic acid having 10-19 carbon atoms and a saturated alcohol having 14-40 carbon atoms or one or more esters of a saturated carboxylic acid having 20-40 carbon atoms and a saturated alcohol having 35-40 carbon atoms in the ratio of approximately 1:1 to approximately 1:5, in particular approximately 1:3.

6. Cosmetic sticks according to Claim 1 or 2, characterized in that the ester, or esters, are selected from amongst the group comprising
hexacosanyl palmitate, octacosanyl palmitate, triacontanyl palmitate, dotriacontanyl palmitate, tetratriacontanyl palmitate, hexacosanyl stearate, octacosanyl stearate, triacontanyl stearate, dotriacontanyl stearate, tetratriacontanyl stearate.

7. Cosmetic sticks according to Claim 1 or 2, characterized in that they contain one or more emulsifiers from amongst the group of the polyglycerol fatty acid esters, in particular polyglyceryl 3-diisostearate.

8. Cosmetic sticks according to Claim 1 or 2, characterized in that they additionally contain, besides the components
(a) beeswax,
(b) octacosanyl stearate and
(c) water
fatty components selected from amongst the group comprising
(d) glycerol tricarboxylic acid esters,
(e) Guerbet alcohols,
(f) myristyl myristate,
(g) jojoba oil and
(h) fractionated coconut oils.

9. Cosmetic sticks according to Claim 1 or 2, characterized in that they additionally contain, besides the components of the group
(a) beeswax,
(b) octacosanyl stearate and
(c) water
and, if appropriate,
fatty components selected from amongst the group comprising
(d) glycerol tricarboxylic acid esters,
(e) Guerbet alcohols,
(f) myristyl myristate,
(g) jojoba oil and
(h) fractionated coconut oils,
substances selected from amongst the group comprising waxes, hydrocarbons, fats, oils, auxiliaries and additives, such as perfume oils, preservatives, pigments, light stabilizers, stabilizers and fat- and/or water-soluble active substances.

10. Process for the preparation of cosmetic sticks according to one of Claims 1-9, characterized in that
(1) beeswax and octacosanyl stearate,
and, if appropriate, one or more emulsifiers selected from amongst the group of the W/O emulsifiers, in particular the polyglycerol fatty acid esters, particularly advantageously polyglyceryl 3-diisostearate,
and fatty components selected from amongst the group of the glycerol tricarboxylic acid esters, Guerbet alcohols, myristyl myristate, jojoba oil and/or related substances and the fractionated coconut oils
are melted and, if appropriate, subjected to steps (2) and (3), i.e.
(2) water is added continuously until the desired water content of the cosmetic stick is achieved,
(3) the resulting mixture is stirred during the addition of water, and
(4) the uniform, stirred mixture is then introduced into moulds and allowed to cool slowly.

## Revendications

1. Bâtons cosmétiques, exempts de cires, graisses ou huiles minérales, contenant
(a) de la cire d'abeille,
(b) des esters de
(ba) un acide carboxylique saturé ayant de 10 à 19 atomes de carbone et
(bb) un alcool saturé ayant de 14 à 40 atomes de carbone,
(c) de l'eau, ainsi que
(d) éventuellement d'autres lipides et/ou des additifs et adjuvants usuels.

2. Bâtons cosmétiques, exempts de cires, graisses ou huiles minérales, contenant
(a) de la cire d'abeille,
(b) des esters de
(ba) un acide carboxylique saturé ayant de 20 à 40 atomes de carbone et
(bb) un alcool saturé ayant de 35 à 40 atomes de carbone,
(c) de l'eau, ainsi que
(d) éventuellement d'autres lipides et/ou des additifs et adjuvants usuels.

3. Bâtons cosmétiques selon la revendication 1, caractérisés en ce qu'ils contiennent
0,5 - 50 % en poids de cire d'abeille
0,5 - 50 % en poids d'un ester d'un acide carboxylique saturé ayant de 10 à 19 atomes de carbone et d'un alcool saturé ayant de 14 à 40 atomes de carbone,
0,1 - 20 % en poids d'eau,
en particulier
2,0 - 20 % en poids de cire d'abeille,
2,0 - 25 % en poids d'un ester d'un acide carboxylique saturé ayant de 10 à 19 atomes de carbone et d'un alcool saturé ayant de 14 à 40 atomes de carbone,
1,0 - 10 % en poids d'eau,
de préférence
3,0 - 10 % en poids de cire d'abeille,
5,0 - 20 % en poids d'un ester d'un acide carboxylique saturé ayant de 10 à 19 atomes de carbone et d'un alcool saturé ayant de 14 à 40 atomes de carbone,
1,0 - 5 % en poids d'eau,
de façon particulièrement préférée,
4,0 - 6,0 % en poids de cire d'abeille,
15,0 - 18,0 % en poids d'un ester d'un acide carboxylique saturé ayant de 10 à 19 atomes de carbone et d'un alcool saturé ayant de 14 à 40 atomes de carbone,
2,0 - 3,0 % en poids d'eau.

4. Bâtons cosmétiques selon la revendication 2, caractérisés en ce qu'ils contiennent
0,5 - 50 % en poids de cire d'abeille
0,5 - 50 % en poids d'un ester d'un acide carboxylique saturé ayant de 20 à 40 atomes de carbone et d'un alcool saturé ayant de 35 à 40 atomes de carbone,
0,1 - 20 % en poids d'eau,
en particulier
2,0 - 20 % en poids de cire d'abeille,
2,0 - 25 % en poids d'un ester d'un acide carboxylique saturé ayant de 20 à 40 atomes de carbone et d'un alcool saturé ayant de 35 à 40 atomes de carbone,
1,0 - 10 % en poids d'eau,
de préférence
3,0 - 10 % en poids de cire d'abeille,
5,0 - 20 % en poids d'un ester d'un acide carboxylique saturé ayant de 20 à 40 atomes de carbone et d'un alcool saturé ayant de 35 à 40 atomes de carbone,
1,0 - 5 % en poids d'eau,
de façon particulièrement préférée,
4,0 - 6,0 % en poids de cire d'abeille,
15,0 - 18,0 % en poids d'un ester d'un acide carboxylique saturé ayant de 10 à 19 atomes de carbone et d'un alcool saturé ayant de 35 à 40 atomes de carbone,
2,0 - 3,0 % en poids d'eau.

5. Bâtons cosmétiques selon la revendication 1 ou 2, caractérisés en ce qu'ils contiennent de la cire d'abeille et un ou plusieurs esters d'un acide caboxylique saturé ayant de 10 à 19 atomes de carbone et d'un alcool saturé ayant de 14 à 40 atomes de carbone, ou un ou plusieurs esters d'un acide carboxylique saturé ayant de 20 à 40 atomes de carbone et d'un alcool saturé ayant de 35 à 40 atomes de carbone, en un rapport allant d'environ 1:1 à environ 1:5, en particulier d'environ 1:3.

6. Bâtons cosmétiques selon la revendication 1 ou 2, caractérisés en ce que le ou les esters sont choisis dans le groupe constitué par le palmitate d'hexacosanyle, le palmitate d'octacosanyle, le palmitate de triacontanyle, le palmitate de dotriacontanyle, le palmitate de tétratriacontanyle, le stéarate d'hexacosanyle, le stéarate d'octacosanyle, le stéarate de triacontanyle, le stéarate de dotriacontanyle, le stéarate de tétratriacontanyle.

7. Bâtons cosmétiques selon la revendication 1 ou 2, caractérisés en ce qu'ils contiennent un ou plusieurs émulsifiants choisi dans le groupe des esters d'acides gras et de polyglycérol, en particulier le 3-diisostéarate de polyglycérol.

8. Bâtons cosmétiques selon la revendication 1 ou 2, caractérisés en ce que, outre les composants
(a) cire d'abeille,
(b) stéarate d'octacosanyle et
(c) eau,
ils contiennent des composants gras choisis dans le groupe constitué par
(d) les tricarboxylates de glycéryle,
(e) les alcools de Guerbet,
(f) le myristate de myristyle,
(g) l'huile de jojoba,
(h) des huiles de coprah fractionnées.

9. Bâtons cosmétiques selon la revendication 1 ou 2, caractérisés en ce que, outre les composants du groupe
(a) cire d'abeille,
(b) stéarate d'octacosanyle et
(c) eau,
et éventuellement
des composants gras choisis dans le groupe constitué par
(d) les tricarboxylates de glycéryle,
(e) les alcools de Guerbet,
(f) le myristate de myristyle,
(g) l'huile de jojoba
(h) des huiles de coprah fractionnées,
ils contiennent des substances choisies parmi des cires, des hydrocarbures, des graisses, des huiles, des adjuvants et des additifs tels que des huiles essentielles, des conservateurs, des pigments colorés, des agents photoprotecteurs, des stabilisants, ainsi que des substances actives hydrosolubles et/ou liposolubles.

10. Procédé pour la fabrication de bâtons cosmétiques selon l'une des revendications 1 à 9, caractérisé en ce que
(1) on fait fondre de la cire d'abeille et du stéarate d'octacosanyle, ainsi qu'éventuellement un ou plusieurs émulsifiants, choisis dans le groupe des émulsifiants E/H, en particulier des polyesters d'acides gras et de glycérol, de façon particulièrement avantageuse le 3-diisostéarate de polyglycérol, ainsi que des composants gras, choisis parmi les tricarboxylates de glycérol, les alcools de Guerbet, le myristate de myristyle, l'huile de jojoba et/ou des substances apparentées et les huiles de coprah fractionnées,
et éventuellement on soumet le mélange aux étapes (2) et (3), à savoir
(2) on y ajoute de l'eau en continu, jusqu'à ce que soit atteinte la teneur désirée en eau du bâton cosmétique,
(3) on délaie pendant l'addition d'eau le mélange résultant,
(4) et on introduit ensuite dans des moules le mélange délayé de façon homogène, et on le laisse lentement refroidir.
